# EUROPÄISCHE PATENTSCHRIFT

(11) **EP 0 499 229 B1**
(45) Veröffentlichungstag und Bekanntmachung des Hinweises auf die Patenterteilung: **14.12.1994**
(21) Anmeldenummer: 92102329.7
(22) Anmeldetag: 12.02.1992
(51) Int. Cl.: C07C 309/15, A61K 7/075

(54) **Alkylethercarbonsäuretauride**
Alkyl ether carboxylic acid taurides
Taurides d'acides alkyl éther carboxyliques

(30) Priorität: 12.02.1991 DE 4104226
(43) Veröffentlichungstag der Anmeldung: 19.08.1992
(73) Patentinhaber: HOECHST AKTIENGESELLSCHAFT, 65926 Frankfurt am Main (DE)
(72) Erfinder: Gerdau, Thomas, Dr., W-6239 Eppstein/Taunus (DE); Reng, Alwin, W-6233 Kelkheim (Taunus) (DE); Kunz, Walter, W-6234 Hattersheim am Main (DE)

(56) Entgegenhaltungen:
- CH-A- 411 221
- DE-A- 3 412 094
- DE-B- 1 197 885
- CHEMICAL ABSTRACTS, vol. 114, no. 7, 18. Februar 1991, Columbus, Ohio, US; abstract no. 61539B, Seite 637

## Beschreibung

Die Erfindung bezieht sich auf Alkylethercarbonsäuretauride, auf ihre Herstellung und ihre Verwendung; Die Herstellung kosmetischer und technischer Reinigungsmittel erfolgt üblicherweise mit anionischen, kationischen, amphoteren und nichtionischen grenzflächenaktiven Substanzen, die allein oder in Form von Kombinationen innerhalb dieser Gruppen als Wirksubstanzen verwendet werden.

Primäre Aufgabe dieser Präparate ist beispielsweise bei kosmetischen Reinigungspräparaten die gezielte Entfernung körpereigener und körperfremder Verunreinigungen der Haut, Haare und Zähne. Bedingt durch die zeitgemäße Tendenz zum täglichen Haarwaschen und Duschen werden in zunehmendem Maße Tenside mit milder, d.h. mit geringer Reinigungswirkung und gleichzeitig guter Haut- und Augenschleimhaut-Verträglichkeit gewünscht.

Die meisten der auf dem Markt befindlichen Tenside, wie Seifen, Alkylsulfate, Alkylethersulfate, Alkylbenzolsulfonate und Alkansulfonate erfüllen diese Forderungen nur in begrenztem Maße. Es wird deshalb versucht, durch alleinigen Einsatz von Spezialtensiden oder durch deren Mischung mit den vorgenannten Tensidklassen die anwendungstechnischen Eigenschaften zu verbessern.

Eine bekannte Tensidklasse mit hervorragenden Eigenschaften sind die Fettsäure-N-methyltauride (DE-B-1 197 885, JP-A-02 218 657). Sie sind charakterisiert durch ein sehr gutes Schäumvermögen in weichem Wasser, gutes Kalkseifendispergiervermögen und eine akzeptable Hautfreundlichkeit. Dennoch erfüllen diese Fettsäure-N-methyltauride nicht in allen Anwendungen die Ansprüche an hochwertige Tenside. So ist beispielsweise die Schaumstabilität in hartem Wasser häufig nicht ausreichend.

Weitere Nachteile sind die schwierige Konsistenzregulierung, d.h. mangelnde Verdickbarkeit in wäßriger Lösung sowohl allein als auch in Kombination mit anderen anionischen Tensiden, wie z.B. Alkylsulfaten oder Alkylethersulfaten und die mäßige Augenschleimhautverträglichkeit, die beispielsweise beim Einsatz dieser Tenside in Haarshampoos eine wesentlichen Rolle spielt.

Es wurde nun gefunden, daß Tauride von Alkylethercarbonsäuren der Formel I

R²-(OC₂H₄)ₙ-O-CH₂-COOH

diese nachteiligen Eigenschaften nicht haben.

Gegenstand der Erfindung sind daher Alkylethercarbonsäuretauride der Formel II

R²-(OC₂H₄)ₙ-O-CH₂-CO-NR¹-C₂H₄-SO₃M

worin n größer als 0 vorzugsweise 1 bis 12, insb. 2 bis 6, R¹ = H, C₁-C₄-Alkyl, R² = C₅-C₂₂-, insb. C₁₀-C₂₂-Alkyl und M = H, Na, K, Ca/2 oder Mg/2 bedeuten. Diese Verbindungen sind oberflächenaktive Stoffe und können überall dort mit Vorteil eingesetzt werden, wo eine Erniedrigung der Oberflächenspannung des Wassers erwünscht oder erforderlich ist. Dies gilt beispielsweise für den Einsatz bei der Herstellung von kosmetischen und technischen Reinigungsmitteln, wie Haarshampoos, Duschbäder, Badepräparate, Zahnpasten, Handgeschirrspülmitteln, Feuerlöschschäumen, Autoshampoos, Textilwaschmitteln, vorallem aber in Präparaten, bei deren Verwendung ein kurz- oder langzeitiger Kontakt mit der menschlichen Haut auftritt.

Als bevorzugte Tenside kommen insbesondere die N-Methyltauride von Alkylethercarbonsäuren in Betracht, d.h. R¹ = Methyl.

Die Verbindungen der Erfindung werden insbesondere in Form ihrer Natrium-, Kalium-, Calcium oder Magnesiumsalze benutzt.

Die Eigenschaften der Alkylethercarbonsäuretauride können durch Wahl des Alkylrestes R² sowie des Oxethylierungsgrades n in weitem Rahmen variiert und den Anforderungen für die jeweilige Anwendung angepaßt werden. Gewöhnlich liegen die erfindungsgemäßen Verbindungen in Form von Mischungen mit verschiedenen Alkylresten R² und verschiedenem Oxethylierungsgrad n vor. R² umfaßt geradkettige und verzweigte Alkylreste, das mit dem Sauerstoffatom verknüpfte C-Atom des Alkylrestes R² ist ein primäres oder sekundäres C-Atom.

Zur Herstellung der erfindungsgemäßen Verbindungen kommen als Ausgangsmaterialien Alkylethercarbonsäuren der Formel I in Frage, die auch kommerziell erhältlich sind, wie z.B. ® Akypo RLM (Hersteller Chem-Y), ® Marlowet 4543 (Hersteller Hüls) oder ® Sandopan D-LW (Hersteller Sandoz). Eine andere Möglichkeit ist die Verwendung von speziellen Alkylethercarbonsäuren, die entweder aus Fettalkoholoxethylaten und Natriumchloracetat oder durch katalytische Oxidation von Fettalkoholoxethylaten zugänglich sind. Unter Umständen kann es wünschenswert sein, Alkylethercarbonsäuren einzusetzen, die aus Fettalkoholoxethylaten mit einer engen Homologenverteilung hergestellt worden sind. Fettalkoholoxethylate mit enger Homologenverteilung erhält man durch Umsetzung von Fettalkoholen mit Ethylenoxid in Gegenwart von Antimonpentahalogeniden als Katalysator (GB-PS 796,508; US-PS 3,359,331).

Die weitere Herstellung zu den erfindungsgemäßen Verbindungen erfolgt durch Umsetzung der Alkylethercarbonsäuren mit Thionylchlorid, Phosphortrichlorid, Phosgen o.ä. zu den entsprechenden Säurechloriden, die wiederum in der üblichen Verfahrensweise unter Schotten-Baumann-Bedingungen mit wäßrigen Tauridlösungen zur Reaktion gebracht werden.

Die erfindungsgemäßen Produkte sind wertvolle anionische oberflächenaktive Stoffe, die vielfach Verwendung finden. So werden sie beispielsweise als Netz-, Schaum- oder Waschmittel verwendet. Sie können als wäßrige Lösung oder als trockene Pulver eingesetzt werden. Im letzteren Fall wird das Wasser beispielsweise durch Abdestillieren oder Trocknen entfernt.

Wenn für besondere Anwendungen das Produkt im wesentlichen salzfrei sein soll, wird das Salz in an sich bekannter Weise, wie z.B. durch Umkehrosmose, entfernt.

Die erfindungsgemäßen Tenside werden im Bedarfsfall in Kombination mit bekannten anionischen , nichtionischen, kationischen und/oder amphoteren Tensiden in vielfältiger Weise angewandt. Dabei können die Alkylethercarbonsäuretauride im Über- oder Unterschuß mit den jeweiligen Tensidklassen kombiniert werden.

Die Erfindung wird durch folgende Beispiele näher erläutert:

### Beispiel 1:

Als Ausgangsprodukt wurde ein Kokosfettalkohol eingesetzt, welches auf übliche Weise mit Antimonpentachlorid als Katalysator mit 2 Mol Ethylenoxid oxethyliert (enge Homologenverteilung) und nach EP-A-0206 054 an einem Platinkontakt zu Cocosalkyl-glykolethercarbonsäure oxidiert wurde. 144,5 g (0,5 Mol) der so hergestellten Säure wurden mit 1 ml Dimethylformamid auf 60 °C erwärmt und mit 77,5 g (0,65 Mol) Thionylchlorid innerhalb einer Stunde versetzt. Nach Beendigung der Gasentwicklung wurde eine weitere Stunde bei 60 °C nachgerührt und leicht siedende Bestandteile im Wasserstrahlvakuum entfernt. Man erhielt 155 g Cocosalkyl-glykolethercarbonsäurechlorid. Das so erhaltene Säurechlorid wurde innerhalb von zwei Stunden zu einer Mischung aus 220 g 37%iger N-Methyltauridnatriumsalzlösung und 250 g Wasser getropft. Durch gleichzeitige Zugabe von Natronlauge wurde der pH im Bereich zwischen 9 und 10 gehalten. Nach beendeter Reaktion wurde eine weitere Stunde nachgerührt und mit Salzsäure auf pH 7 eingestellt. Man erhielt 695 g einer wäßrigen Lösung von Cocosalkyl-glykolethercarbonsäure-N-methyltauridnatriumsalz. Der Gehalt an waschaktiver Substanz betrug 25,1 % nach Epton. Die kritische Mizellkonzentration (CMC) liegt bei 0,26 g/l.

### Beispiel 2:

In analoger Weise wurde Cocosalkyl-diglykolethercarbonsäure-N-methyltauridnatriumsalzlösung hergestellt. Der Gehalt an waschaktiver Substanz betrug 24,7 %. Die kritische Mizellkonzentration ist 0,19 g/l.

### Beispiel 3:

In gleicher Weise wurde auf Basis einer Mischung von synthetischen Alkoholen mit C₁₂-C₁₅-Alkylgruppen eine C₁₂-C₁₅-alkyl-triglykolethercarbonsäure-N-methyltaurid-Natriumsalzlösung hergestellt. Der Gehalt an waschaktiver Substanz betrug 24,3 % nach Epton. Die kritische Mizellkonzentration ist 0,12 g/l.

Die nachfolgenden Anwendungsbeispiele zeigen die breiten Einsatzmöglichkeiten der Alkylethercarbonsäuretauride in Reinigungsmitteln für kosmetische Anwendungen, für den Haushalt und für technische Zwecke. Die Mengen- und Prozentangaben in den Beispielen beziehen sich, sofern nichts anderes erwähnt, jeweils auf das Gewicht.

### Beispiel 4:

| Haarshampoo | |
|---|---|
| Alkylethercarbonsäuretaurid hergestellt nach Beispiel 1 | 60,00 % |
| Laurylalkohol + 3 Mol Ethylenoxid | 3,00 % |
| Parfümöl | 0,30 % |
| Natriumchlorid | 0,30 % |
| Wasser | ad 100,00 % |

### Beispiel 5:

| Schaumbad | |
|---|---|
| Alkylethercarbonsäuretaurid, hergestellt nach Beispiel 3 | 68,00 % |
| Laurylalkohol + 3 Mol Ethylenoxid | 3,00 % |
| Parfümöl | 0,50 % |
| Wasser | ad 100,00 % |

### Beispiel 6:

| Shampoo für jeden Tag | |
|---|---|
| Alkylethercarbonsäuretaurid, hergestellt nach Beispiel 1 | 50,00 % |
| Laurylalkohol + 3 Mol Ethylenoxid | 3,00 % |
| Acylamidodiglykolethersulfat-Magnesiumsalz | 8,00 % |
| Natriumchlorid | 1,50 % |
| Wasser | ad 100,00 % |

### Beispiel 7:

| Shampoo für trockene Haare | |
|---|---|
| Alkylethercarbonsäuretaurid, hergestellt nach Beispiel 2 | 57,00 % |
| Polyethylenglykol-6000-distearat | 3,20 % |
| Wasser | ad 100,00 % |

### Beispiel 8:

| Antischuppenshampoo | |
|---|---|
| Alkylethercarbonsäuretaurid, hergestellt nach Beispiel 1 | 18.00 % |
| Lauryldiglykolethersulfat-Natriumsalz (28 %ig) | 38,00 % |
| Natriumchlorid | 2,00 % |
| Antischuppenwirkstoff ®Octopirox (Hoechst AG) | 0,75 % |
| Wasser | ad 100,00 % |

Beispiel 9:

| Antischuppenshampoo | |
|---|---|
| Alkylethercarbonsäuretaurid, hergestellt nach Beispiel 3 | 20,00 % |
| Lauryltriglykolethersulfat-Natriumsalz (28 %ig) | 40,00 % |
| Natriumchlorid | 1,80 % |
| Antischuppenwirkstoff ®Octopirox (Hoechst AG) | 0,50 % |
| Wasser | ad 100,00 % |

### Beispiel 10:

| Duschbad | |
|---|---|
| Alkylethercarbonsäuretaurid, hergestellt nach Beispiel 1 | 18,00 % |
| Laurylsulfat-Natriumsalz (34%ig) | 30,00 % |
| Cocosfettsäurediethanolamid | 3,00 % |
| Wasser | ad 100,00 % |

### Beispiel 11:

| Duschbad | |
|---|---|
| Alkylethercarbonsäuretaurid, hergestellt nach Beispiel 3 | 20,00 % |
| Laurylsulfat-Triethanolaminsalz (40 %ig) | 25,00 % |
| Cocosfettsäurediethanolamid | 3,00 % |
| Parfümöl | 0,50 % |
| Wasser | ad 100,00 % |

### Beispiel 12:

| Flüssiges Handreinigungsmittel | |
|---|---|
| Alkylethercarbonssäuretaurid, hergestellt nach Beispiel 1 | 12,00 % |
| Alkyldiglykolethersulfat-Natriumsalz (28 %ig) | 32,00 % |
| sekundäres Alkansulfonat-Natriumsalz (60 %ig) | 5,00 % |
| Cocosfettsäurediethanolamid | 3,00 % |
| Natriumchlorid | 0,90 % |
| Wasser | ad 100,00 % |

### Beispiel 13:

| Babyshampoo | |
|---|---|
| Alkylethercarbonsäuremethyltaurid, hergestellt nach Beispiel 3 | 45,00 % |
| Eiweißfettsäurekondensat, mittlere Molmasse 500 | 3,00 % |
| Cocosamphocarboxiglycinat | 5,00 % |
| Laurylalkohol + 3 Mol Ethylenoxid | 3,00 % |
| Natriumchlorid | 2,00 % |
| Wasser | ad 100,00 % |

Die vorgenannten Anwendungsbeispiele zeigen die Einsatzmöglichkeiten der Alkylethercarbonsäuremethyltauride in kosmetischen Reinigungsmitteln. In ähnlicher Weise lassen sich naturgemäß auch Reinigungsmittel für den Haushalt, Geschirrspülmittel, Waschmittel, Schutzpasten, Syndetseifen und die verschiedenen Reinigungsmittel für technische Anwendungsgebiete herstellen.

Auch die praxisüblichen Zusätze wie Farbstoffe, Perlglanzmittel, Parfümöle, Wirkstoffe, Antioxidantien, Chelatbildner, Konservierungsmittel und dgl. können in der bekannten Weise in den Reinigungspräperaten verwendet werden.

In den nachfolgenden Versuchen werden verschiedene Eigenschaften der Alkylethercarbonsäuretauride, hergestellt nach Beispiel 1, 2 und 3, mit denen von handelsüblichen anionischen Tensiden gegenübergestellt.

### 1. Schäumvermögen

Das Schäumvermögen wurde nach ROSS-MILES (F.J. Gohlke: Die Bestimmung des Schaumvermögens von Detergentien nach "ROSS-MILES", Parfümerie und Kosmetik 45, 359-363, 1964) in Wasser von 0° und 20° dH bei einer Temperatur von 43 °C bestimmt. Als Vergleichssubstanz diente das Cocosfettsäuremethyltaurid-Natriumsalz (Handelsprodukt). Die Schaumhöhe ist jeweils in mm angegeben, WAS bedeutet Waschaktive Substanz.

| Alkylethercarbonsäuremethyltaurid gemäß | | | | | | | | |
|---|---|---|---|---|---|---|---|---|
| % WAS | Beispiel 1 | | Beispiel 2 | | Beispiel 3 | | Cocosfettsäuremethyltaurid-Natriumsalz | |
| | 0°dH | 20°dH | 0°dH | 20°dH | 0°dH | 20°dH | 0°dH | 20°dH |
| 1,0 | 230 | 240 | 230 | 240 | 240 | 240 | 220 | 210 |
| 0,1 | 220 | 220 | 210 | 210 | 220 | 220 | 210 | 200 |
| 0,03 | 200 | 200 | 190 | 190 | 200 | 200 | 170 | 170 |
| 0,006 | 130 | 130 | 130 | 120 | 130 | 120 | 100 | 60 |
| 0,002 | 90 | 50 | 80 | 40 | 60 | 50 | 40 | 35 |

Die Werte zeigen eine deutliche Überlegenheit der erfindungsgemäßen Tenside hinsichtlich der Schaumstabilität gegenüber dem handelsüblichen Vergleichsprodukt.

### 2. Konsistenzgebende Eigenschaften

Speziell für den Eisatz in der Praxis zur Herstellung mittel- bis hochviskoser Verdickpräperate spielen die konsistenzgebenden Eigenschaften von Tensiden eine wichtige Rolle.
Zur Prüfung dieses Verhaltens wird eine 10 % WAS-haltige Mischung, bestehend aus 7 % Lauryldiglykolethersulfat-Natriumsalz und 3 % Prüfprodukt hergestellt. Dieser Mischung wird in steigender Konzentration Natriumchlorid zugesetzt, und die resultierende Viskosität mit dem Brookfield-Viskosimeter RV3 gemessen.
Die erhaltenen Werte in m Pas für diese Mischung mit Alkylethercarbonsäuremethyltauriden, hergestellt gem. Beispiel 1, 2 und 3, sind in der folgenden Tabelle festgehalten.

| Alkylethercarbonsäuremethyltaurid, gemäß | | | |
|---|---|---|---|
| NaCl % | Beispiel 1 | Beispiel 2 | Beispiel 3 |
| | (3 Gew.-Tl. mit 7 Gew.-Tl. Lauryldiglykolethersulfatsalz) | | |
| 7,0 | 42 400 | 32 300 | 38 200 |
| 8,0 | 55 200 | 38 600 | 34 300 |
| 9,0 | 30 550 | 21 000 | 27 000 |

Die obigen Werte veranschaulichen das günstige anwendungstechn. Verhalten der erfindungsgemäßen Tenside im Hinblick auf ihre konsistenzgebenden Eigenschaften.

### 3. Zein-Test

Der Zein-Test (s. A.K. Reng et al., Erfahrungen mit Invitro-Methoden zur Charakterisierung kosmetischer Tenside, Parfümerie und Kosmetik 68, 771-778, 1987) gibt orientierende Hinweise über die Hautverträglichkeit von Tensiden.
Hohe Zeinwerte, d.h. über 500 mg Stickstoff/100 ml deuten auf stark solubilisierende Tenside mit ungünstigem dermatologischen Verhalten hin. Zeinwerte unter 200 hingegen gelten orientierend für sogenannte milde Tenside.

Die erfindungsgemäßen Tenside zeigen folgende Werte (Zeinwerte in mg Stickstoff/100 ml).

| Alkylethercarbonsäuremethyltaurid gemäß | | | | |
|---|---|---|---|---|
| | Beispiel 1 | Beispiel 2 | Beispiel 3 | Laurylsulfat-Natriumsalz |
| Zeinwert | 169 | 205 | 172 | 834 |

Aus den obigen Ergebnissen ist zu ersehen, daß die Verbindungsklasse unter der Grenze von 200 mg Stickstoff/100 ml liegt und damit als mild betrachtet werden kann. Die Vergleichssubstanz Laurylsulfat-Natriumsalz zeigte einen Zeinwert von 834 und ist damit als stark irritierend einzuordnen.

### 4. Hämoglobin-Denaturierung

Die Hämoglobin-Denaturierung (nach Pape und Hoppe, Proceedings 2 ND World Tenside Congress 1988, Vol. 4) gibt Hinweise über das Verhalten von Tensiden auf der Humanhaut. Durch Komplexbildung des Tensides mit Globin verändert sich dessen Struktur, und die Farbänderung des Hämoglobins läßt sich spektral photometrisch messen. Natriumlaurylsulfat wird dabei als 100 % denaturierend festgelegt. Die erfindungsgemäßen Substanzen ergaben Denaturierungswerte zwischen 40 und 50 % und können somit als gut verträglich bezeichnet werden.

## Patentansprüche (Patentansprüche für folgende(n) Vertragsstaat(en): AT, BE, CH, DE, DK, FR, GB, IT, LI, NL, SE)

1. Alkylethercarbonsäuretauride der Formel
R²-(OC₂H₄)ₙ-O-CH₂-CO-NR¹-C₂H₄-SO₃M,
in der n größer als O, insbesondere 1 bis 12,
und
R¹ = H oder C₁-C₄-Alkyl, insbesondere Methyl,
R² = C₅-C₂₂-Alkyl, insbesondere C₁₀-C₂₂-Alkyl, und
M = H, Na, K, Ca/2 oder Mg/2 bedeuten.

2. Verfahren zur Herstellung der Verbindung nach Anspruch 1, wobei man von den Alkylethercarbonsäuren der Formel
R²-(OC₂H₄)ₙ-O-CH₂-COOH
ausgeht, diese in das entsprechende Säurechlorid überführt und das Säurechlorid mit wäßriger Tauridlösung der Formel
NHR¹-C₂H₄-SO₃M
unter den Bedingungen der Schotten-Baumann-Reaktion zum gewünschten Produkt umsetzt, wobei R¹, R², M und n die in Anspruch 1 angegebene Bedeutung haben.

3. Verwendung der Verbindungen des Anspruchs 1 als anionische oberflächenaktive Stoffe, insbesondere zur Herstellung von Reinigungsmitteln.

4. Reinigungsmittel, insbesondere für kosmetische Anwendungen, für Anwendungen im Haushalt oder für technische Zwecke, enthaltend mindestens eine Verbindung des Anspruchs 1.

## Patentansprüche (Patentansprüche für folgende(n) Vertragsstaat(en): ES)

1. Verfahren zur Herstellung von Alkylethercarbonsäuretauriden der Formel
R²-(OC₂H₄)ₙ-O-CH₂-CO-NR¹-C₂H₄-SO₃M,
in der n größer als O, insbesondere 1 bis 12,
und
R¹ = H oder C₁-C₄-Alkyl, insbesondere Methyl,
R² = C₅-C₂₂-Alkyl, insbesondere C₁₀-C₂₂-Alkyl, und
M = H, Na, K, Ca/2 oder Mg/2 bedeuten,
wobei man von den Alkylethercarbonsäuren der Formel
R²-(OC₂H₄)ₙ-O-CH₂-COOH
ausgeht, diese in das entsprechende Säurechlorid überführt und das Säurechlorid mit wäßriger Tauridlösung der Formel
NHR¹-C₂H₄-SO₃M
unter den Bedingungen der Schotten-Baumann-Reaktion zum gewünschten Produkt umsetzt, wobei R¹, R², M und n die vorstehend angegebene Bedeutung haben.

2. Verwendung der Verbindungen des Anspruchs 1 als anionische oberflächenaktive Stoffe, insbesondere zur Herstellung von Reinigungsmitteln.

3. Reinigungsmittel, insbesondere für kosmetische Anwendungen, für Anwendungen im Haushalt oder für technische Zwecke, enthaltend mindestens eine Verbindung des Anspruchs 1.

## Claims (Claims for the following Contracting State(s): AT, BE, CH, DE, DK, FR, GB, IT, LI, NL, SE)

1. An alkyl ether carboxylic acid tauride of the formula
R²-(OC₂H₄)ₙ-O-CH₂-CO-NR¹-C₂H₄-SO₃M,
in which n is greater than 0, especially 1 to 12, R¹ = H or C₁-C₄ alkyl, especially methyl, R² = C₅-C₂₂ alkyl, especially C₁₀-C₂₂ alkyl, and M = H, Na, K, Ca/2 or Mg/2.

2. A process for the preparation of a compound as claimed in claim 1, wherein the starting material is an alkyl ether carboxylic acid of the formula
R²-(OC₂H₄)ₙ-O-CH₂-COOH,
this is converted to the corresponding acid chloride and the acid chloride is reacted with an aqueous solution of a tauride of the formula
NHR¹-C₂H₄-SO₃M,
under the conditions of the Schotten-Baumann reaction, to give the desired product, R¹, R², M and n being as defined in claim 1.

3. The use of a compound of claim 1 as an anionic surface- active substance, especially for the preparation of cleaning agents.

4. A cleaning agent, especially for cosmetic applications, for household applications or for technical purposes, containing at least one compound of claim 1.

## Claims (Claims for the following Contracting State(s): ES)

1. A process for the preparation of an alkyl ether carboxylic acid tauride of the formula
R²-(OC₂H₄)ₙ-O-CH₂-CO-NR¹-C₂H₄-SO₃M,
in which n is greater than 0, especially 1 to 12, R¹ = H or C₁-C₄ alkyl, especially methyl, R² = C₅-C₂₂ alkyl, especially C₁₀-C₂₂ alkyl, and M = H, Na, K, Ca/2 or Mg/2 wherein the starting material is an alkyl ether carboxylic acid of the formula
R²-(OC₂H₄)ₙ-O-CH₂-COOH,
this is converted to the corresponding acid chloride and the acid chloride is reacted with an aqueous solution of a tauride of the formula
NHR¹-C₂H₄-SO₃M,
under the conditions of the Schotten-Baumann reaction, to give the desired product, R¹, R², M and n being as defined above.

2. The use of a compound of claim 1 as an anionic surface- active substance, especially for the preparation of cleaning agents.

3. A cleaning agent, especially for cosmetic applications, for household applications or for technical purposes, containing at least one compound of claim 1.

## Revendications (Revendications pour l'(les) Etat(s) contractant(s) suivant(s): AT, BE, CH, DE, DK, FR, GB, IT, LI, NL, SE)

1. Taurides d'acides alkyléthercarboxyliques de formule
R²-(OC₂H₄)ₙ-O-CH₂-CO-NR¹-C₂H₄-SO₃M,
dans laquelle n est supérieur à 0, plus particulièrement 1 à 12,
et
R¹ = H ou alkyle en C₁-C₄, plus particulièrement le méthyle,
R² = alkyle en C₅-C₂₂, plus particulièrement alkyle en C₁₀-C₂₂, et
M = H, Na, K, Ca/2 ou Mg/2,

2. Procédé pour la préparation du composé selon la revendication 1, en partant des acides alkyléthercarboxyliques de formule
R²-(OC₂H₄)ₙ-O-CH₂-COOH
que l'on transforme en chlorure d'acide correspondant et on fait réagir le chlorure d'acide avec la solution aqueuse de tauride de formule :
NHR¹-C₂H₄-SO₃M
dans les conditions de la réaction de Schotten-Baumann pour obtenir le produit voulu, R¹, R², M et n ayant les significations données citées dans la revendication 1.

3. Utilisation des composés selon la revendication 1 en tant qu'agents de surface anioniques, plus particulièrement pour la préparation de produits de nettoyage.

4. Produits de nettoyage, plus particulièrement pour des utilisations en cosmétique, en ménage ou en industrie, contenant au moins un composé de la revendication 1.

## Revendications (Revendications pour l'(les) Etat(s) contractant(s) suivant(s): ES)

1. Taurides d'acides alkyléthercarboxyliques de formule :
R²-(OC₂H₄)ₙ-O-CH₂-CO-NR¹-C₂H₄-SO₃M,
dans laquelle n est supérieur à 0, plus particulièrement 1 à 12,
et
R¹ = H ou alkyle en C₁-C₄, plus particulièrement le méthyle,
R² = alkyle en C₅-C₂₂, plus particulièrement alkyle en C₁₀-C₂₂, et
M = H, Na, K, Ca/2 ou Mg/2.
en partant d'un acide alkyléthercarboxylique de formule :
R²-(OC₂H₄)ₙ-O-CH₂-COOH
que l'on transforme en chlorure d'acide correspondant et on fait réagir le chlorure d'acide avec la solution aqueuse de tauride de formule :
NHR¹-C₂H₄-SO₃M
dans les conditions de la réaction de Schotten-Baumann pour obtenir le produit voulu, R¹, R², M et n ayant les significations données dans la revendication 1.

2. Utilisation des composés selon la revendication 1 en tant qu'agents de surface anioniques, plus particulièrement pour la préparation de produits de nettoyage.

3. Produits de nettoyage, plus particulièrement pour des utilisations en cosmétique, en ménage ou en industrie, contenant au moins un composé de la revendication 1.
